(19) Europäisches Patentamt
European Patent Office
Office européen des brevets

(11) Numéro de publication: **0 121 489**
**B1**

(12) # FASCICULE DE BREVET EUROPÉEN

(45) Date de publication du fascicule du brevet: **26.11.86**

(51) Int. Cl.⁴: **C 07 D 311/30**, A 61 K 31/35

(21) Numéro de dépôt: **84450005.8**

(22) Date de dépôt: **07.03.84**

(54) **Nouveaux dérivés de la tétrahydroxy-3', 4', 5, 7 flavone, leur méthode de préparation et leur emploi thérapeutique.**

(30) Priorité: **01.04.83 FR 8305567**

(43) Date de publication de la demande:
**10.10.84 Bulletin 84/41**

(45) Mention de la délivrance du brevet:
**26.11.86 Bulletin 86/48**

(84) Etats contractants désignés:
**BE CH DE GB IT LI LU NL**

(56) Documents cité:
**EP-A-0 019 081**
**US-A-3 661 890**

**CHEMICAL ABSTRACTS, vol. 70, 1969, page 346, no. 3756g, Columbus, Ohio, US; S.E. DREWES et al.: "Isolation of 3-methoxyfisetin from Acacia mearnsi"**

(73) Titulaire: **SOCIETE CORTIAL S.A., 7 rue de l'Armorique, F-75015 Paris (FR)**

(72) Inventeur: **Creuzet, Marie- Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Guichard, Françoise, Les Cèdres Parc des Tourelles Rue St Elisabeth, F-33200 Bordeaux (FR)**
Inventeur: **Mosser, Jacqueline, Rue du Colonel Rozanoff, F-33160 Saint- Médard- en- Jalles (FR)**
Inventeur: **Prat, Gisèle, Hameau de Noailles - Villa 18, F-33400 Talence (FR)**
Inventeur: **Pontagnier, Henri, 21 rue Edouard Vaillant, F-33600 Pessac (FR)**

(74) Mandataire: **Tajan, Marie- Thérèse, LABORATOIRES SARGET Avenue du Président JF Kennedy, F-33701 Mérignac (FR)**

**EP 0 121 489 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouvelles hydroxy-3 flavones diversement substituées sur l'oxygène en position 3, leur méthode de préparation et leur application thérapeutique.

Les nouveaux produits faisant l'objet de la présente invention ont pour formule générale

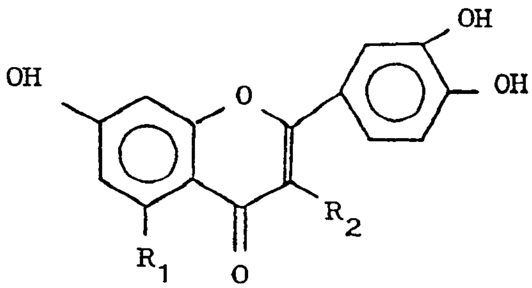

(I)

dans laquelle $R_1$ = H ou OH et $R_2$ = alcoxy inférieur de C2 à C6, cycloalcoxy de C5 à C8, méthanesulfonyloxy ou paratoluènesulfonyloxy sous réserve que $R_1$ soit différent de OH si $R_2$ = alcoxy inférieur de C2 à C4.

Les produits tels que $R_1$ = OH sont dérivés de la quercétine et les produits tels que $R_1$ = H sont dérivés de la fisétine.

On connaissait déjà quelques éthers méthyliques, et en particulier O-méthyl-3, de flavonols. Un certain nombre sont des dérivés naturels. La Société Biosedra avait breveté sous le N° FR-A-2 088 127 la pentabenzylquercétine utilisée en thérapeutique dans les indications classiques des flavonoïdes (inhibition de l'hyperperméabilité et réduction de la fragilité capillaire).

Dans les publications C.A.70, n° 3756g, p. 346 (1969) et US-A-3.661.890 des dérivés de flavone sont également divulgués. De plus les dérivés de flavone connus de US-A-3.661.890 ont une activité antibactérienne.

La Société Hoffman-la-Roche a déposé le 9.4.1980 sous priorité anglaise du 10.4.1979 une demande européenne n° 80 101 894 6 publieé sous le n° 19 081 décrivant des alcoxy-3 dihydroxy-5,7 flavones à activité antivirale et en particulier des produits répondant à la formule générale I avec $R_1$ = OH et $R_2$ = alcoxy inférieur de C1 à C4 et de préférence égal à méthoxy ou éthoxy. Ces produits bien que compris dans la formule générale telle qu'elle est présentée dans ce brevet européen ne sont toutefois pas décrits dans les exemples illustrant l'invention.

Nous avons maintenant découvert que les flavones de formule I avec $R_1$ = H ou OH et $R_2$ = alcoxy inférieur de C2 à C6, cycloalcoxy de C5 à C8, méthanesulfonyloxy ou paratoluènesulfonyloxy sous réserve que $R_1$ soit différent de OH si $R_2$ = alcoxy inférieur de C2 à C4, présentent des propriétés inhibitrices de l'aldose réductase permettant leur emploi dans la prevention des complications, oculaires et nerveuses en particulier, du diabète. Ces nouveaux produits sont également utiles en tant qu'hypoglycémiants et hypolipidémiants.

Les produits faisant l'objet de la présente invention sont préparés de la façon suivante:

- Les produits de formule I tels que $R_2$ = méthanesulfonyloxy ou paratoluènesulfonyloxy sont préparés à partir des glycosides de flavones tels que $R_1$ = H ou OH et $R_2$ = 0-glycosyl. Les groupements phénoliques libres du glycoside sont bloqués par exemple sous forme d'esters benzoïques; l'aglycone est obtenue par action d'acide chlorhydrique concentré. Le groupement OH-3 du flavonol est ensuite estérifié par le chlorure de mésityle ou le chlorure de l'acide paratoluènesulfonique. Les groupements benzoates sont éliminés par traitement à la soude.

- Les produits de formule 1 tels que $R_1$ = H ou OH et $R_2$ = alcoxy inférieur de C2 à C6 ou cycloalcoxy de C5 à C8, sous réserve que $R_1$ soit différent de OH si $R_2$ = alcoxy inférieur de C2 à C4, sont préparés par action d'un dérivé de formule

$R_2CH_2CN$ et de

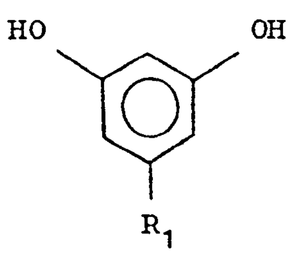

en présence d'acide chlorhydrique conduisant à

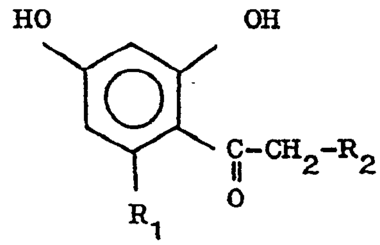

Ce dérivé réagit avec l'anhydride de l'acide dibenzyloxy-3,4 benzoïque pour conduire à un produit de formule

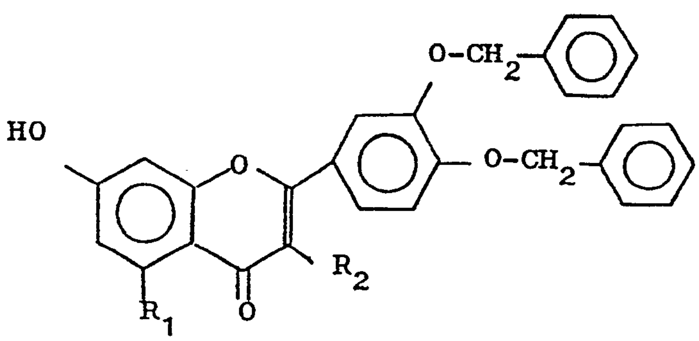

Cet éther est transformé en produit de formule I par hydrogénolyse en présence par exemple de palladium/BaSO4.

La présente invention va être illustrée par les exemples suivants.

**Exemple 1**

Synthèse de la cyclohexyloxy-3 tétrahydroxy-5,7,3',4' flavone; produit de formule 1 avec $R_1$ = OH, $R_2$ =

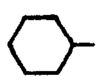 -O-; nom de code COR 1983.

**Synthèse du cyclohexyloxyacétonitrile**

Dans un réacteur de Grignard de 500 $cm^3$ on introduit 100g de cyclohexanol, 40 g de trioxane, 200 $cm^3$ de toluène. Ce mélange est porté à 0°C sous agitation. On fait barboter de l'acide chlorhydrique gazeux pendant quatre heures. L'eau formée est décantée.

Le chlorométhoxycyclohexane est séché puis distillé. Eb = 75°C sous 27 millibars. Un mélange composé de 62 g de ce dérivé et 56 g de cyanure cuivreux est chauffé au reflux à 120°C pendant 4 heures. Le cyclohexyloxyacétonitrile est distillé. Eb 60-70°C sous 0,014 millibars. Rendement 75 %.

**Synthèse du cyclohexylglycoloyl-2 phloroglucinol**

On dissout dans 500 $cm^3$ d'éther anhydre 20 g de cyclohexyloxyacétonitrile et 18 g de phloroglucinol anhydre. On fait barboter de l'acide chlorhydrique gazeux pendant 4 heures à 0°C et on maintient sous agitation une nuit à 0°C. On filtre. Le chlorhydrate de cétimine est repris dans 250 $cm^3$ d'ethanol bouillant et 250 $cm^3$ d'eau chaude sont ajoutés. Le mélange est chauffé au reflux pendant trois heures. La réaction est suivie en

chromatographie sur couches minces. Après refroidissement le cyclohexyl-glycoloyl-2 phloroglucinol se sépare sous forme d'huile puis cristallise. Il est recristallisé dans un mélange alcool/eau, repris dans du chloroforme puis précipité dans de l'éther de pétrole. Rendement 60 %.

**Synthèse de l'anhydride de l'acide dibenzyloxy-3,4 benzoïque**

Dans un réacteur de Grignard de 2 litres on introduit 138 g de dihydroxy-3,4 benzaldéhyde, 138 g de carbonate de potassium et un litre de diméthylformamide (DMF). Le mélange est porté au reflux sous agitation. 253 g de chlorométhylbenzène sont ajoutés goutte à goutte. Le mélange est maintenu sous agitation au reflux pendant 6 heures. Après refroidissement, le sel ayant précipité est filtré et lavé avec un peu de DMF. Le milieu réactionnel est versé par petites quantités dans 15 litres d'eau glacée sous bonne agitation. Le dibenzyloxy-3,4 benzaldéhyde précipite sous forme pâteuse puis cristallise. Le solide est filtré puis repris dans du méthanol bouillant. Après une filtration à chaud, on refroidit la solution méthanolique sous agitation et le précipité formé est filtré, lavé avec du méthanol glacé, de l'éther éthylique, puis séché. Rendement 77 %.

Dans un ballon de 4 litres le mélange composé de 1,5 litre d'eau, 700 $cm^3$ de pyridine, 245 g de dibenzyloxy-3,4 benzaldehyde est chauffé au reflux. 162 g de permanganate de potassium sont ajoutés peu à peu. Le mélange est chauffé au reflux pendant une heure puis filtré à chaud et lavé avec un litre d'eau bouillante. Le premier filtrat est concentré pour éliminer la pyridine. Après acidification par $H_2SO_4$ au 1/2 de l'ensemble des filtrats, le précipité est filtré puis lavé avec de l'eau et séché. Rendement 85 %.

Le mélange constitué de un litre de benzène, 130 g d'acide dibenzyloxy-3,4 benzoïque et 45 $cm^3$ de chlorure de thionyle est chauffe pendant deux heures au reflux et sous agitation. Après évaporation et reprise à l'éther, le chlorure d'acide est filtré et lavé à l'éther froid. Rendement 90 %.

Dans un ballon muni d'un desséchant le mélange constitué par 1,5 1 d'éther anhydre, 290 $cm^3$ de pyridine, 100,2 g d'acide dibenzyloxy-3,4 benzoïque et 105,7 g du chlorure de l'acide dibenzyloxy-3,4 benzoïque est agité pendant 24 heures à température ambiante. Le mélange est ensuite versé dans 8 litres d'eau glacée. Après 45 mn d'agitation le précipité est filtré, lavé avec un litre d'acide chlorhydrique 0,1N glacé, un litre de $Na_2CO_3$0,1N glacé puis un litre d'eau glacée. Le solide est solubilisé dans $CHCl_3$; on décante, sèche et évapore et l'anhydride est recristallisé dans l'acétate d'éthyle. Rendement 88 %.

**Synthèse de la dibenzyloxy-3',4' dihydroxy-5,7 cyclohexyloxy-3 flavone**

Dans un ballon de 2 litres on introduit 13,3 g de cyclohexylglycoloyl-2 phloroglucinol préparé selon 1b, 130 g d'anhydride préparé selon 1c et 28 $cm^3$ de triéthylamine. Le mélange réactionnel est plongé dans un bain à 170° C. Il est chauffé 3 heures sous agitation puis refroidi à 100° C. Après addition de 1 litre de méthanol puis 60 $cm^3$ de potasse à 60 % il est chauffé 45 mn au reflux. Après refroidissement à 30° C on ajoute 1,5 litre d'eau froide. Le précipité formé est filtré. La phase aqueuse est extraite 3 fois à l'éther. La solution est amenée à pH 10 avec un courant de $CO_2$, extraite trois fois à l'acétate d'éthyle. La phase organique est séchée, évaporée. Le résidu est repris dans l'éther. Rendement 64 %.

**Synthèse de la cyclohexyloxy-3 tétahydroxy-5,7,3',4' flavone**

On introduit dans un ballon de deux litres 27 g de dibenzyloxy-3',4' dihydroxy 5,7 cyclohexyloxy-3 flavone, 500 $cm^3$ d'acétone, 500 $cm^3$ de méthanol, 8 spatules de palladium sur $BaSO_4$ à 5 %. Le mélange est agité sous atmosphère d'hydrogène pendant 3 heures. La réaction est suivie en chromatographie sur couches minces. Après filtration sur millipores on évapore la solution. Rendement 98 %.

Spectre de RMN dans le DMSOD6: 0,8-2,3 ppm, 10 protons, massif complexe, $CH_2$; 4,0-4,6 ppm, 1 proton, massif complexe, H-C-O-; 6,2 ppm, 1 proton, doublet, H-6; 6,4ppm, 1 proton, doublet, H-8; 6,9 ppm, 1 proton, doublet, H-5'; 7,3-7,8 ppm, 2 protons, massif complexe, H-2' + H-6'; 9,9 ppm, 3 protons, dôme, OH-7 + OH-3' + OH-4', échangeables avec $D_20$; 12,8 ppm,1 proton, singulet, OH-5 échangeable avec $D_20$.

PF = 260-270° C (banc Kofler).

Microanalyse élémentaire: calculé C 65,62 %; H 5,24 %; 0 29,13%

Trouvé C 65,63 %; H 5,30 %; 0 29,40

On prépare de la même façon l'éthoxy-3 trihydroxy-7,3',4' flavone, produit de la formule I avec $R_1$ = H et $R_2$ = $OC_2H_5$, nom de code COR 1987

Spectre de RMN dans le DMSOD6: 1,3 ppm, 3 protons, triplet, $CH_3$; 4,1 ppm, 2 protons, quadruplet, $CH_2$; 6,7-8,1 ppm, 6 protons, massif complexe, protons aromatiques; 9,9 ppm, 3 protons, pic large, OH, échangeable avec $D_20$.

PF = 254° C (appareil Mettler).

**Exemple 2**

Synthèse de la mèthanesulfonyloxy-3 tétrahydroxy-3',4',5,7 flavone; produit de formule I avec $R_1$ = OH, $R_2$ = $CH_3$-$SO_2$-0; nom de code COR 1988.

**Synthèse de tétrabenzoyloxy-3',4',5,7 flavone**

800 g de carbonate de potassium sont dissous dans 7 litres d'eau. 100 g de rutine sont ajoutés sous agitation. Quand le mélange est devenu limpide, on ajoute 400 cm$^3$ de chlorure de benzoyle en deux fois. Le mélange est maintenu sous agitation vigoureuse pendant deux heures. Le solide est filtré et lavé a neutralité. Il est séché sous vide sur $P_20_5$, puis mis en suspension dans 3 à 4 litres d'éther èthylique; le mélange est agité vigoureusement. Le solide est filtré et cette manipulation est renouvelée une seconde fois. 205 g de tétrabenzoate de rutine brut ainsi obtenus sont dissous dans 1,3 litre d'éthanol bouillant. 400 cm$^3$ d'acide chlorhydrique concentré sont ajoutés en une fois. Le mélange est agité en maintenant au reflux de l'éthanol pendant une heure. La phase alcoolique est séparée du solide qui a précipité en masse visqueuse. Le solide est broyé dans un mortier et lavé à l'eau puis dissous dans du chloroforme. Après lavage à l'eau, séchage et évaporation, le résidu est repris dans 700 cm d'acétone. Le mélange est porté au reflux sous agitation, filtré à chaud. Le solide est lavé avec un peu d'acétone. Rendement 28,2 % (par rapport à la rutine).

**Synthèse de la méthanesulfonyloxy-3 tétrahydroxy-3',4',5,7 flavone**

49 g de tétrabenzoate de quercétine sont dissous dans 180 cm$^3$ de pyridine. 10 cm$^3$ de chlorure de mésityle sont ajoutés. Le mélange est maintenu sous agitation pendant une heure à température ambiante. Une nouvelle quantité de 10 cm de chlorure de mésityle est rajoutée et l'agitation est maintenue à température ambiante pendant trois heures. Le mélange est versé dans l'eau glacée sous agitation. Après filtration le solide est lavé à l'eau, dissous dans du chloroforme. La phase chloroformique est séchée et évaporée. 600 cm$^3$ d'acétone sont rajoutés au résidu d'évaporation. Le mélange est porté au reflux sous agitation puis filtré à chaud. Rendement 54,3 %.

8 g de tetrabenzoate de méthanesulfonyloxy-3 flavone sont dissous dans un mélange de 1,5 litres d'acétone et de méthanol bouillant et sous argon. 2 g de soude dissous dans du méthanol sont ajoutés au mélange réactionnel. Après avoir vérifié par chromatographie en couches minces que la réaction est totale, le mélange est refroidi et le pH est amené vers 9 à l'aide d'un courant de $CO_2$. Les solvants sont évaporés. De l'acétate d'éthyle est ajouté au résidu. Le mélange est mis sous agitation, puis le solvant est décanté. Le résidu est solubilisé dans l'eau. La solution est neutralisée en utilisant du $CO_2$ et le précipité est filtré. Il est séché sous vide sur $P_20_5$. Rendement 38 %.

Spectre de RMN dans DMSOD6: 3,5 ppm, 3 protons, singulet, $CH_3$; 6,3 ppm, 1 proton, doublet, H-6; 6,5 ppm, 1 proton, 1 doublet, H-8; 7,0 ppm, 1 proton, 1 doublet, H-5'; 7,3-7,7 ppm, 2 protons, massif complexe, H-2' + H-6'; 10,0 ppm, 3 protons, dôme, OH-7 + OH-3' + OH-4', échangeables avec $D_20$; 12,1 ppm, 1 proton, dôme, OH-5, échangeable avec $D_20$.

PF = 233° C. (appareil Mettler)

On prépare de la même façon la paratoluènesulfonyloxy-3 tétrahydroxy-5,7,3',4' flavone, produit de formule 1 avec $R_1$ = OH et $R_2$ = paratoluénesulfonyloxy; nom de code COR 1990.

Les propriétés pharmacologiques des produits faisant l'objet de la présente invention sont exposées ci-après.

L'intérêt des produit de la présente invention dans le traitement et la prophylaxie des complications du diabète a été mis en évidence in vitro par la détermination de l'activité inhibitrice de l'aldose réductase et in vivo dans le modèle de la neuropathie induite par la streptozotocine.

**Inhibition de l'aldose reductase in vitro**

L'enzyme est extraite de cristallins de boeufs par la méthode décrite par Hayman S. et Kinoshita J.H. (J. Biol. Chem. 1965, 240, 2, 877). Le pourcentage d'inhibition de la capacité de l'enzyme à réduire le glycéraldéhyde en glycérol sous l'effet du produit à tester est déterminé par dosage spectométrique de la quantité de NADPH réagissant suivant la méthode décrite par Hayman et Kinoshita. Nous donnons ci-après entre parenthèses après chaque produit la valeur du log 1/CI50 où CI50 représente la concentration exprimée en mole/l entrainant 50% d'inhibition de l'activité enzymatique: COR 1983 (5, 8), COR 1987 (6, 3), COR 1988 (6, 8), COR 1990 (5, 5). Ces valeurs sont à comparer à la valeur correspondante pour la quercétine (5, 0);

**Neuropathie à la streptozotocine**

4 lots de 10 rats pesant environ 200 g sont traités de J -2 à J +3 par un julep gommeux du produit à tester. A JO les animaux reçoivent une injection IP de 100 mg/kg de streptozotocine dissoute dans un tampon citrate. La glycémie est déterminée 24 heures et 3 jours après l'injection de l'agent diabétogène. Les rats sont sacrifiés le 3° jour après l'injection de streptozotocine et les nerfs sciatiques sont prélevés pour déterminer les taux de sorbitol et d'inositol. Testé dans ces conditions le COR 1988 entraine une diminution du taux de sorbitol qui passe de 0,819 ± 0,215 mg/kg chez les témoins à 0,608 ± 0,049 mg/kg chez les animaux traités par 50 mg/kg et à 0,482 ± 0.091 mg/kg chez les animaux traités par 100 mg/kg. De même le taux d'inositol passe de 2,174 ± 0,481 mg/kg chez les témoins à 1,593 ± 0,109 mg/kg chez les animaux traités par 25 mg/kg, à 1,430 ± 0,104 mg/kg chez les animaux traité par 50 mg/kg et à 1,402 ± 0,130 mg/kg chez les animaux traités par 100 mg/kg. Les taux de sorbitol et d'inositol après traitement sont comparés aux taux témoins par le test U de Mann et Whitney (Schwartz D. Méthodes statistiques à l'usage des médecins et biologistes, Ed. Flammarion, Paris 1963). Les résultats obtenus sont significatifs avec $p < 1\%$. La glycémie des animaux témoins est de 4,72 ± 0,60 g/l à J3. Elle passe à 3,93 ± 0,82 g/l chez les animaux traités par 50 mg/kg de COR 1988 et à 3,61 ± 0,73 g/l chez les animaux traités par 100 mg/kg. Ces abaissements de glycémie sont significatifs. La toxicité des produits de la présente invention est déterminée chez la souris.

Le COR 1983 administré par voie orale en solution dans de l'eau en présence de gomme à 6 % ne détermine aucune mortalité à la dose de 1 g/kg. Administré par voie intrapéritonéale en solution dans le "Tween" à 5 % il ne provoque aucune mortalité jusqu'à 100 mg/kg et entraine 10 % de mortalité à la dose de 200 mg/kg.

Le COR 1988 administré par voie orale en solution dans l'eau en présence de gomme à 6 % ne provoque aucune mortalité jusqu'à la dose de 3 g/kg.

Les produits faisant l'objet de la présente invention sont utiles en tant qu'hypolipidémiants. Ainsi le COR 1983 administré 2 fois à la dose de 400 mg/kg par voie orale à 20 heures d'intervalle chez la souris hypercholestérolémique entraine 24 % de diminution de la cholestérolémie.

Compte tenu de leurs propriétés jointes à une faible toxicité, les produits selon la présente invention sont utiles en thérapeutique humaine et vétérinaire, par exemple dans le traitement et la prévention des complications oculaires et nerveuses du diabète ou dans le traitement du diabète et des hyperlipidémies. Les produits faisant l'objet de la présente invention peuvent être utilisés seuls ou associés à des antidiabétiques. Ils seront administrés, associés aux véhicules et excipients appropriés, par voie orale sous forme de dragées, comprimés, sirop, ampoules buvables, par voie rectale sous forme de suppositoires, par voie parentérale en injections sous cutanées, intramusculaires, intraveineuses, par voie topique sous forme de pommades ou gels. Ils peuvent également entrer dans des compositions à usage ophtalmique sous forme de collyres ou de pommades. Les doses administrées varieront selon l'indication et le sujet de 5 à 500 mg/j en 2 à 6 prises pour la voie orale, de 5 à 500 mg/j en une ou deux prises pour la voie rectale, de 0,5 à 50 mg par injection pour les voies parentérales.

**Revendications**

1.- Produits de formule générale

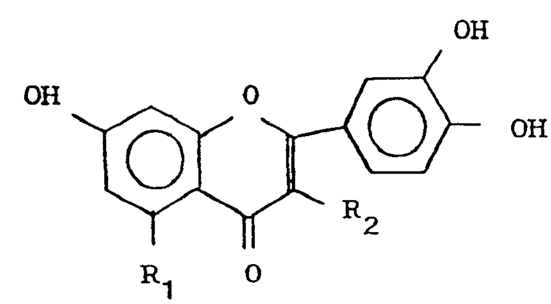

avec $R_1$ = H ou OH et $R_2$ = alcoxy inférieur de C2 à C6, cycloalcoxy de C5 à C8, méthanesulfonyloxy ou paratoluènesulfonyloxy sous réserve que $R_1$ soit différent de OH si $R_2$ = alcoxy inférieur de C2 à C4.

2 - Procédé de préparation des produits selon la revendication 1 et tels que $R_2$ = méthanesulfonyloxy ou paratoluènesulfonyloxy caractérisé en ce que l'on bloque les groupements phénoliques libres d'un glycoside de flavone de formule

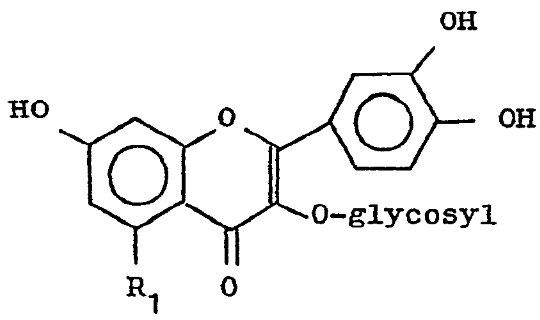

avec $R_1$ = H ou OH, par exemple sous forme d'ester benzoïque, en ce que le groupement OH-3 est libéré par action d'acide chlorhydrique concentré, puis estérifié par le chlorure de mésityle ou le chlorure de l'acide paratoluènesulfonique, et en ce que les groupements benzoates sont éliminés par traitement à la soude.

3 - Procédé de préparation des produits selon la revendication 1 et tels que $R_1$ = H ou OH et $R_2$ = alcoxy inférieur de C2 à C6 ou cycloalcoxy de C5 à C8 sous réserve que $R_1$ soit différent de OH si $R_2$ = alcoxy inférieur de C2 à C4, caractérisé en ce que l'on fait réagir un dérivé de formule $R_2CH_2CN$ avec

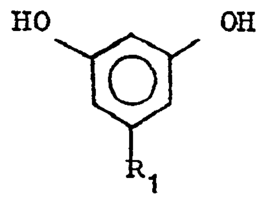

en présence d'acide chlorhydrique, en ce que le dérivé ainsi obtenu réagit avec l'anhydride de l'acide dibenzyloxy-3,4 benzoïque, et en ce que la flavone ainsi préparée est débenzylée par hydrogénolyse en présence, par exemple, de palladium/BaSO4.

4 - Médicament caractérisé en ce que le principe actif est constitué par au moins un produit selon la revendicatiom 1.

5 - Médicament selon la revendication 4 utile en thérapeutique préventive et curative des complications du diabète.

6 - Médicament selon la revendication 4 utile en tant qu'hypolipidémiant ou hypoglycémiant.

7 - Composition pharmaceutique ou vétérinaire caractérisée en ce qu'elle contient à titre de principe actif au moins un produit selon la revendication 1 en association avec un véhicule pharmaceutique ou un excipient approprié.

**Patentansprüche**

1. Verbindungen der allgemeinen Formel

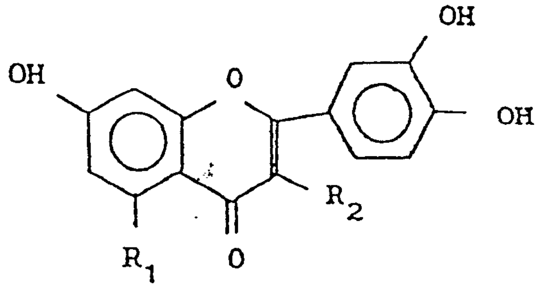

in der $R_1$ = H oder OH und $R_2$ = niedriges $C_2$- bis $C_6$-Alkoxy, $C_5$- bis $C_8$-Cycloalkoxy, Methansulfonyloxy oder p-Toluolsulfonyloxy bedeuten, mit der Einschränkung, daß $R_1$ nicht OH ist, wenn $R_2$ = niedriges $C_2$- bis $C_4$-Alkoxy ist.

2. Verfahren zur Herstellung der Verbindungen nach Anspruch 1, soweit $R_2$ = Methansulfonyloxy oder p-Toluolsulfonyloxy ist, dadurch gekennzeichnet, daß man die freien phenolischen Gruppen eines Flavonglycosids der Formel

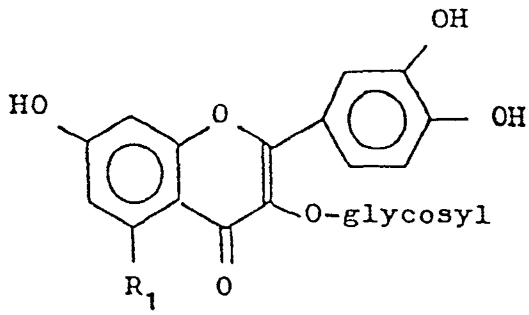

in der $R_1$ = H oder OH ist, z.B. in Form eines Benzoesäureesters blockiert, daß man die Gruppe OH-3 durch Einwirkung von konzentrierter Salzsäure freisetzt und anschließend mit Mesylchlorid oder p-Toluolsulfonylchlorid verestert, und daß man die Benzoesäureestergruppen durch Behandlung mit Soda eliminiert.

3. Verfahren zur Herstellung der Verbindungen nach Anspruch 1, soweit $R_1$ = H oder OH und $R_2$ = niedriges $C_2$- bis $C_6$-Alkoxy oder $C_5$- bis $C_8$-Cycloalkoxy bedeuten, mit der Einschränkung, daß $R_1$ nicht OH ist, wenn $R_2$ = niedriges $C_2$- bis $C_4$-Alkoxy ist, dadurch gekennzeichnet, daß man ein Derivat der Formel $R_2CH_2CN$ mit

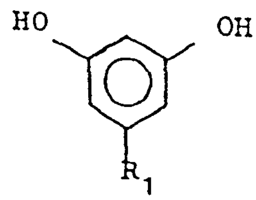

in Gegenwart von Salzsäure umsetzt, daß man das so erhaltene Derivat mit dem Anhydrid der Dibenzyloxy-3,4-benzoesäure reagieren läßt und daß man das so erhaltene Flavon durch Hydrogenolyse in Gegenwart von z.B. Palladium/$BaSO_4$ debenzyliert.

4. Arzneimittel, dadurch gekennzeichnet, daß es als Wirkstoff mindestens eine der Verbindungen nach Anspruch 1 enthält.

5. Arzneimittel nach Anspruch 4 zur Verwendung in der präventiven und kurativen Therapie von Komplikationen der Diabetes.

6. Arzneimittel nach Anspruch 4 zur Verwendung als hypolipidämisches oder hypoglykämisches Mittel.

7. Pharmazeutische oder veterinärmedizinische Zubereitung, dadurch gekennzeichnet, daß sie als Wirkstoff mindestens eine Verbindung nach Anspruch 1 zusammen mit einem pharmazeutischen Träger oder geeigneten Exzipienten enthält.

**Claims**

1. Products with the general formula

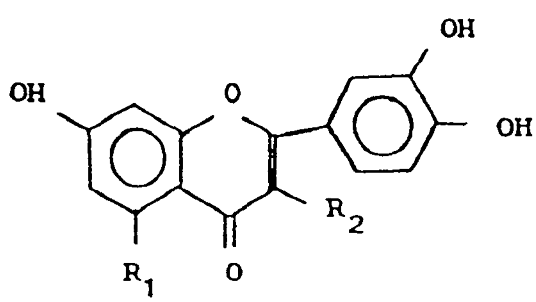

with $R_1$ = H or OH and $R_2$ = lower alkoxy from C2 to C6, cycloalkoxy from C5 to C8, methane sulfonyloxy or paratoluene sulfonyloxy, with the condition that $R_1$ be different from OH when $R_2$ = lower alkoxy from C2 to C4

2. Method for preparing products as defined in claim 1 and such that $R_2$ = methane sulfonyloxy or paratoluene sulfonyloxy, which comprises blocking the free phenolic groups from a flavon glycoside with as formula

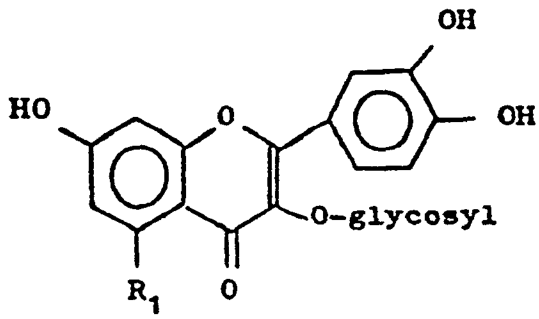

with $R_1$ = H or OH, for example in the form of benzoic ester, releasing the OH-3 group under the action of concentrated hydrochloric acid, then esterifying same with mesityl chloride or paratoluene sulfonic acid chloride, and removing the benzoate groups by soda-processing.

3. Method for preparing products as defined in claim I and such that $R_1$ = H or OH and $R_2$ = lower alkoxy from C2 to C6 or cycloalkoxy from C5 to C8, with the condition that $R_1$ be different from OH when $R_2$ = lower alkoxy from C2 to C4, which comprises reacting a derivative with as formula $R_2CH_2CN$, together with

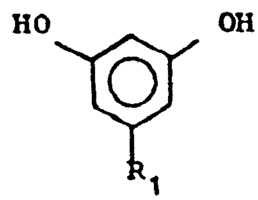

in the presence of hydrochloric acid, reacting the resulting derivative with dibenzyloxy-3,4 benzoic acid anhydrid, and un-benzylizing the resulting flavon by hydrogenolysis in the presence for example, of palladium/$BaSO_4$.

4. Medicament, in which the active constituent is comprised of at least one product as defined in claim 1.

5. Medicament as defined in claim 4, useful for preventive and curative therapy of diabetes involvements.

6. Medicament as defined in claim 4, useful as hypolipoidic agent or hypoglycohaemic agent.

7. Pharmaceutic or veterinary composition, which contains as active constituent, at least one product as defined in claim 1 associated with a suitable pharmaceutic vehicle or excipient.